# EUROPÄISCHE PATENTSCHRIFT

(11) **EP 3 993 741 B1**
(45) Veröffentlichungstag und Bekanntmachung des Hinweises auf die Patenterteilung: **23.04.2025**
(21) Anmeldenummer: 20736955.4
(22) Anmeldetag: 30.06.2020
(51) Int. Cl.: A61F 2/66, A61F 2/70, A61F 2/76

(54) **PROTHESENEINRICHTUNG FÜR EINE UNTERE EXTREMITÄT, EINSTELLEINRICHTUNG FÜR EINE PROTHESENEINRICHTUNG SOWIE VERFAHREN ZUM MANUELLEN EINSTELLEN**
PROSTHETIC DEVICE FOR A LOWER EXTREMITY, ADJUSTING DEVICE FOR A PROSTHETIC DEVICE, AND METHOD FOR MANUAL ADJUSTMENT
DISPOSITIF PROTHÉTIQUE DESTINÉ À UNE EXTRÉMITÉ INFÉRIEURE, DISPOSITIF DE RÉGLAGE DESTINÉ À UN DISPOSITIF PROTHÉTIQUE AINSI QUE PROCÉDÉ DE RÉGLAGE MANUEL

(30) Priorität: 04.07.2019 DE 102019118118
(43) Veröffentlichungstag der Anmeldung: 11.05.2022
(73) Patentinhaber: Ottobock SE & Co. KGaA, 37115 Duderstadt (DE)
(72) Erfinder: GLORIEUX, Dries, 3920 Lommel (BE); SCHNEIDER, Gregory, Salt Lake City, Utah 84103 (US); MEJIA NINO, Juan-Pablo, 2340 Mödling (AT); SCHWARTZ, Miclas, 37083 Göttingen (DE); KALTENBORN, Sven, 37115 Duderstadt (DE)
(74) Vertreter: Gramm, Lins & Partner Patent- und Rechtsanwälte PartGmbB
(86) Internationale Anmeldenummer: PCT/EP2020/068367
(87) Internationale Veröffentlichungsnummer: WO 2021/001353

(56) Entgegenhaltungen:
- EP-A1- 3 035 895
- WO-A1-2018/085014
- DE-A1- 102014 010 938

## Beschreibung

Die Erfindung betrifft eine Protheseneinrichtung für eine untere Extremität mit einem Prothesenfuß und einem an dem Prothesenfuß befestigten Unterschenkelteil sowie eine Einrichtung zum manuellen Einstellen einer Orientierung des Unterschenkelteils relativ zu dem Prothesenfuß. Die Erfindung betrifft ebenfalls eine Einstelleinrichtung zum manuellen Einstellen einer Orientierung eines Unterschenkelteils relativ zu einem Prothesenfuß, einer Protheseneinrichtung, einer unteren Extremität sowie ein Verfahren zum manuellen Einstellen einer Orientierung eines Unterschenkelteils, einer Protheseneinrichtung, einer unteren Extremität relativ zu einem an dem Unterschenkelteil befestigten Prothesenfuß, wobei an der Protheseneinrichtung eine Einstelleinrichtung mit einem Inertialwinkelsensor angeordnet ist, der die Orientierung des Unterschenkelteils im Raum detektiert und mit einer Ausgabeeinrichtung gekoppelt ist.

Prothesen ersetzen nicht oder nicht mehr vorhandene Gliedmaßen, wobei in der Regel angestrebt wird, dass die Prothese nicht nur die Form, sondern auch zumindest einen Teil der Funktion der Gliedmaßen ersetzt. Protheseneinrichtungen der unteren Extremität weisen ein Fußteil auf, den Prothesenfuß, der an dem Patienten festgelegt ist. Sofern der Unterschenkel oder ein Teil des Unterschenkels noch vorhanden ist, kann eine Festlegung des Prothesenfußes an dem jeweiligen Stumpf über einen Unterschenkelschaft erfolgen. Der Unterschenkelschaft kann auf verschiedene Arten und Weisen an dem Stumpf festgelegt werden, beispielsweise mit einem Prothesenliner und einer Saugschaft-Technologie. Ist das natürliche Kniegelenk nicht mehr vorhanden, erfolgt die Festlegung der Protheseneinrichtung in der Regel an einem Oberschenkelstumpf über einen Oberschenkelschaft. An dem Oberschenkelschaft ist dann ein Prothesenkniegelenk festgelegt, das ein Unterschenkelrohr oder ein Unterschenkelteil aufweist, um den Prothesenfuß mit dem Prothesenkniegelenk zu koppeln. An oder in dem Unterschenkelteil können Dämpfereinrichtungen, Verstelleinrichtung sowie Sensoren und Steuerungseinrichtungen beispielsweise zur Steuerung der Dämpfereinrichtung zur Beeinflussung des Prothesenkniegelenks angeordnet sein.

Der Prothesenfuß kann gelenkig und motorisch angetrieben an dem Unterschenkelteil gelagert sein, um den Patienten bei der jeweils vorgesehenen Bewegung zu unterstützen. Die Koordination der Bewegungen in dem Kniegelenk und dem Knöchelgelenk sind bei einer solchen Ausgestaltung sehr aufwendig, darüber hinaus benötigt der jeweilige Antrieb viel Platz und ist vergleichsweise schwer. In der einfachsten Ausführungsform eines Prothesenfußes ist dieser als gelenkloser Prothesenfuß ausgebildet und wird - einmal ausgerichtet - dauerhaft an dem Unterschenkelteil festgelegt. Schwierig ist hierbei eine Anpassung der Ausrichtung des Prothesenfußes an unterschiedliche Absatzhöhen, wenn der Patient einen Schuhwechsel vornimmt. Weiterhin sind verschwenkbar um ein Knöchelgelenk gelagerte Prothesenfüße bekannt, die eine passive Dämpfereinrichtung aufweisen, um eine Dorsalflexion oder Plantarflexion zu beeinflussen. Weiterhin existieren Prothesenfüße mit einem Fußteil und einem proximalen Anschlussmittel, das verschwenkbar mit dem Fußball verbunden ist. Über eine Verstelleinrichtung ist das Fußteil relativ zu dem Anschlussmittel verstellbar. Der Verstelleinrichtung kann zumindest ein Positionssensor zugeordnet sein, der mit einem Signalerzeugungselement gekoppelt ist, das in Abhängigkeit von dem Signal des Positionssensors ein Signal über das Erreichen der Position des Fußteils ausgibt. Der Positionssensor ermittelt die relative Position des Fußteils zu dem Anschlussmittel oder dem daran befestigten Unterschenkelteil. Alternativ ermittelt der Positionssensor die Raumlage des Fußteils während eines Einstellvorganges. Ein solcher Prothesenfuß ist aus der DE 10 2014 010 938 A1 bekannt. Dadurch ist es möglich, dass Absatzhöheneinstellungen wiedergefunden werden können.

Die WO 2018/085014 A1 betrifft ein Verfahren und ein Steuerungssystem für Prothesenfüße, bei dem mehrere Beschleunigungssensoren zur Erfassung von Sensordaten eingesetzt werden. Diese Sensordaten werden gefiltert und ausgewertet, um ein Referenzsignal zu erzeugen, um eine Bewegung einer Prothese zu steuern, die nicht gangbezogen ist. Solche Bewegungen sind beispielsweise das Gleiten des Fußes über dem Boden oder das Beine Kreuzen während des Sitzens, das Stehen oder die Gewichtsverlagerung während des Stehens.

Die WO 2015/024612 A1 betrifft ein Verfahren zur Steuerung eines künstlichen Kniegelenks, an dem eine Unterschenkelkomponente angeordnet und dem eine Widerstandseinrichtung zugeordnet ist, über die der Beugewiderstand in Abhängigkeit von Sensordaten verändert wird. Dabei wird eine Linearbeschleunigung der Unterschenkelkomponente ermittelt und mit zumindest einem Schwellwert verglichen. Bei Erreichen eines Schwellwertes der Linearbeschleunigung der Unterschenkelkomponente wird der Beugewiderstand verändert. Ein Inertialwinkelsensor kann an der Unterschenkelkomponente angeordnet sein, um einen Absolutwinkel der Unterschenkelkomponente zu messen.

Nachteilig an der im Stand der Technik vorgeschlagenen Einrichtung ist, dass die richtige Position für jeden Schuh einzeln eingestellt werden muss, da nur der Relativwinkel zwischen Prothesenfuß und Unterschenkel ermittelt wird. Für eine neue Absatzhöhe muss daher zunächst die korrekte Einstellung ermittelt und als Referenz eingespeichert werden. Zudem muss der Nutzer aus einer Vielzahl an Referenzsignalen das zu der jeweiligen Absatzhöhe passende Signal identifizieren, was zu Fehleinstellungen führen kann.

Aufgabe der vorliegenden Erfindung ist es daher, eine Protheseneinrichtung, eine Einstelleinrichtung sowie ein Verfahren zum Einstellen einer Orientierung eines Unterschenkelteils einer Protheseneinrichtung bereitzustellen, die oder das einfach anzuwenden ist und dem Anwender eine sichere Rückmeldung über den korrekten Prothesenaufbau gibt.

Erfindungsgemäß wird diese Aufgabe durch eine Protheseneinrichtung mit den Merkmalen des Hauptanspruches und eine Einstelleinrichtung sowie ein Verfahren mit den Merkmalen der nebengeordneten Ansprüche gelöst. Vorteilhafte Ausgestaltungen und Weiterbildungen der Erfindung sind in den Unteransprüchen, der Beschreibung den Figuren offenbart.

Die Protheseneinrichtung für eine untere Extremität mit einem Prothesenfuß und einem an dem Prothesenfuß befestigten Unterschenkelteil sowie eine Einrichtung zum manuellen Einstellen einer Orientierung des Unterschenkelteils relativ zu dem Prothesenfuß sieht vor, dass an der Protheseneinrichtung ein Inertialwinkelsensor angeordnet ist, der die Orientierung des Unterschenkelteils im Raum detektiert und mit einer Ausgabeeinrichtung gekoppelt ist, die die Orientierung des Unterschenkelteils im Raum oder das Erreichen einer vorab festgelegten Orientierung für einen Nutzer erkennbar mit einem Ausgabesignal ausgibt. Die Ausgabeeinrichtung gibt bevorzugt die festgelegte Orientierung des Unterschenkelteils im Raum für einen Nutzer erkennbar aus, beispielsweise optisch, akustisch oder taktil und vermittelt so eine Rückkopplung oder ein Feedback über die Orientierung des Unterschenkelteils im Raum. Die Ausgabe kann qualitativ und/oder quantitativ erfolgen oder aber durch ein Signal angeben, dass eine einmal eingespeicherte Orientierung des Unterschenkelteils im Raum erreicht ist. Statt die Orientierung des Prothesenfußes im Raum zu messen und abzugleichen, ob diese Orientierung der gewünschten Orientierung entspricht, wird die Raumlage des Unterschenkelteils detektiert und nicht die relative Position des Unterschenkelteils zu dem Prothesenfuß. Mit einer solchen Protheseneinrichtung ist es möglich, nach der Montage eines verstellbaren Prothesenfußes an dem Unterschenkelteil die Protheseneinrichtung durch einen Orthopädietechniker optimal einzustellen, also einen optimalen Prothesenaufbau zu gewährleisten. Der Prothesenaufbau ist die Positionierung und Orientierung der jeweiligen Prothesenkomponenten zueinander. Der Prothesenaufbau wird für jeden Patienten individuell eingestellt und trägt maßgeblich zur Funktionsfähigkeit und Akzeptanz der Protheseneinrichtung durch den Patienten bei. Ist der Prothesenaufbau einmal korrekt eingestellt, kann der Patient bei der Verwendung eines anderen Schuhmodells mit einer abweichenden Absatzhöhe und einer abweichenden Sohlensteifigkeit den optimalen Prothesenaufbau mit der erfindungsgemäßen Protheseneinrichtung einfach wiederfinden. Durch Verstellung des Prothesenfußes relativ zu dem Unterteil bis zum Erreichen der eingespeicherten Standardwinkels oder Referenzwinkels des Unterteils im Raum wird die durch den Orthopädietechniker eingestellte Optimalstellung einfach aufgefunden, der Prothesenfuß kann in dieser Stellung nach einem Schuhwechsel oder dergleichen an dem Unterschenkelteil fixiert werden. Die Kombination aus Bestimmung des Unterschenkelwinkels mittels Inertialsensor oder Inertialsensoren und einer Ausgabeeinrichtung erlaubt es, die Absatzhöhe für beliebige Prothesenfüße, die über eine Einrichtung zum manuellen Einstellen einer Orientierung des Unterschenkelteils verfügen, einzustellen. Das Einstellen einer Orientierung des Unterschenkelteils erfolgt dabei von dem Entsperren des Gelenkes über das Verstellen des Winkels bis hin zum erneuten Sperren komplett manuell. Es wird entsprechend kein aufwändiges mechatronisches Knöchelgelenk benötigt. Dem Patienten wird vielmehr für jedes verstellbare Knöchelgelenk eine einfache und zuverlässige Möglichkeit geboten, die richtige Einstellung für verschiedene Absatzhöhen aufzufinden. Die Orientierung im Raum ist insbesondere die Orientierung des Unterschenkelteils relativ zu der Schwerkraftrichtung. Befindet sich der Unterschenkel beispielsweise innerhalb der Sagittalebene wird die Orientierung des Unterschenkelteils durch die Vorwärtsneigung oder Rückwärtsneigung in Gegenrichtung ausgehend von der Lotlinie definiert. Wenn der Nutzer der Protheseneinrichtung den Prothesenfuß entsperrt, insbesondere manuell entsperrt, kann der Prothesenfuß relativ zu dem Unterschenkelteil bewegt werden. Dies geschieht vorteilhafter Weise wenn der Prothesenfuß aufgesetzt ist und das Unterschenkelteil innerhalb der Sagittalebene verschwenkt wird, beispielsweise um eine Schwenkachse im Bereich des Knöchelgelenks. Bei Erreichen der korrekten Position des Unterschenkelteils wird ein für den Nutzer wahrnehmbares Signal ausgegeben.

Eine Weiterbildung der Erfindung sieht vor, dass der Inertialwinkelsensor und die Ausgabeeinrichtung als ein Modul zusammengefasst in der Protheseneinrichtung eingebaut oder lösbar daran befestigt sind. Dadurch ist es möglich, die Einstelleinrichtung nachträglich an einer Protheseneinrichtung anzubringen oder eine Nachrüstung vorzunehmen und ursprünglich nicht dafür ausgelegte Protheseneinrichtungen mit dem Modul auszurüsten. Grundsätzlich ist es vorgesehen, dass der Inertialwinkelsensor an einer beliebigen Stelle am Unterschenkelteil sitzt, beispielsweise weit distal, um die Raumlage möglichst unverfälscht zu erfassen. Bevorzugt befindet sich der Inertialsensor proximal des Prothesenfußes und proximal des Knöchelgelenks, sodass wertvoller Bauraum im Prothesenfuß eingespart werden kann. Die Ausgabeeinrichtung kann an einer leicht zugänglichen oder leichter wahrnehmbaren Stelle angeordnet sein, beispielsweise an einem Unterschenkelschaft, einem Oberschenkelschaft oder an einem separaten Ort, unabhängig von der Protheseneinrichtung. Die Ausgabeeinrichtung kann bei einer Ausgestaltung als separates Element als Anhänger an einem Schlüsselbund, in einer Tasche, als Armband oder als App in einem Mobiltelefon ausgeführt sein, sodass der Nutzer das Feedback möglichst bequem erhalten kann. Der Inertialwinkelsensor ist dann mit der Ausgabeeinrichtung bevorzugt kabellos, per Funk oder einer ähnlichen Datenübertragungsmethode gekoppelt. Bei einer Ausgestaltung als Modul, insbesondere nachrüstbares Modul, sind der Inertialwinkelsensor und die Ausgabeeinrichtung dauerhaft, insbesondere per Kabel miteinander verbunden. Bei einer getrennten Anordnung kann die Ausgabeeinrichtung nur bei Bedarf mit dem Inertialwinkelsensor gekoppelt sein. Der Inertialwinkelsensor ist dann mit einer Sendeeinrichtung, die auch als Teil einer Steuerungseinrichtung für die übrige Protheseneinrichtung ausgebildet sein kann, gekoppelt, über die die Ausgabeeinrichtung mit dem entsprechenden Signal über die Position des Unterschenkelteils im Raum versorgt wird. Sowohl der Inertialwinkelsensor als auch die Ausgabeeinrichtung sind bevorzugt mit einer Steuerungseinrichtung gekoppelt, in der die Sensordaten ausgewertet werden. In der Steuerungseinrichtung ist beispielsweise ein Computer und eine Speichereinrichtung vorhanden, um die Sensordaten auszuwerten, einen Abgleich mit einem Referenzwinkel im Raum durchzuführen und ein Signal auszugeben.

Der Inertialwinkelsensor kann bevorzugt an dem Unterschenkelteil angeordnet sein. Alternativ dazu ist vorgesehen, dass der Inertialwinkelsensor an einem proximal dazu angeordneten Element der Protheseneinrichtung befestigt ist, beispielsweise an einem Oberteil eines Prothesenkniegelenkes oder an einem Oberschenkelschaft. Aus der Information über die Raumlage des proximalen Elementes in Verbindung mit einem Winkelsensor, der die Stellung des Unterschenkelteils zum Oberschenkelschaft misst, kann die Raumlage des Unterschenkelteils errechnet werden. Alternativ oder ergänzend kann die Ermittlung des Unterschenkelwinkels bei einem vorher definierten Relativwinkel von Oberschenkel zu Unterschenkel erfolgen, sodass kein Winkelsensor benötigt wird. Dazu könnte vorgesehen sein, dass der Anwender die Einstellung beispielsweise immer bei vollständig gestrecktem Knie durchführt oder durchführen muss.

Eine Weiterbildung der Erfindung sieht vor, dass ein Belastungssensor an der Protheseneinrichtung angeordnet und mit der Ausgabeeinrichtung dergestalt gekoppelt ist, dass das Ausgabesignal mit einer detektierten Belastung ausgegeben wird. Die Orientierung des Unterschenkelteils kann sich bei unterschiedlichen Belastungen, insbesondere bei unterschiedlichen Axialbelastungen verändern. Bei einem Schuhwechsel können aufgrund unterschiedlicher Sohlensteifigkeiten und Sohlengeometrien unterschiedliche Unterschenkelorientierungen und damit jeweils ein unterschiedlicher Prothesenaufbau auftreten. Über den Belastungssensor kann sichergestellt werden, dass die Einstellung bei immer der gleichen Belastung, z.B. Axialbelastung erfolgt, wodurch ein gleichbleibender Prothesenaufbau gewährleistet wird. Der Belastungssensor kann als Axialkraftsensor, Drucksensor oder Momentensensor ausgebildet sein und beispielsweise in dem Unterschenkelteil, in einer Verbindungseinrichtung von dem Prothesenfuß zu dem Unterschenkelteil, an einem Gelenk oder an dem Prothesenfuß angeordnet sein.

Die Ausgabeeinrichtung ist bevorzugt zur Ausgabe eines optischen, akustischen und/oder taktilen Ausgabesignals ausgebildet und kann entweder die momentane Raumlage des Unterschenkelteils, insbesondere in Sagittalebene, die Abweichung in einer bestimmten Richtung zu der eingegebenen und eingespeicherten Orientierung und/oder das Erreichen der vorgegebenen Orientierung anzeigen. Die Ausgabeeinrichtung kann auch mit der Einrichtung zum manuellen Einstellen einer Orientierung des Unterschenkelteils verbunden oder in diese integriert sein. In diesem Fall entriegelt der Nutzer die Verstelleinrichtung und bewegt den Prothesenfuß manuell bis die Referenzposition erreicht ist. Bei Erreichen der Referenzposition wird ein Signal ausgegeben, wodurch der Anwender ein Feedback darüber erhält, dass die korrekte Einstellung gefunden wurde.

Bevorzugt ist der Prothesenfuß in der Sagittalebene verschwenkbar gelagert, um Absatzhöhenänderungen auszugleichen. Sofern auch eine Raumlage in der Frontalebene erfasst wird, kann beispielsweise ein Warnsignal abgegeben werden, wenn die Abweichung von einem vorher eingestellten Wert zu groß ist.

Eine Weiterbildung der Erfindung sieht vor, dass eine Abschalteinrichtung den Inertialwinkelsensor und/oder die Ausgabeeinheit oder die Verbindung von dem Inertialwinkelsensor zu der Ausgabeeinheit nach dem Erreichen der vorab festgelegten Orientierung abschaltet, sodass Energie gespart werden kann. Die Ausgabeeinheit arbeitet bevorzugt nur dann, wenn eine Einstellung und Anpassung an einen neuen Schuh erfolgen soll. Hierzu kann die Protheseneinrichtung in einen Einstellmodus gebracht werden, beispielsweise über ein Eingabefeld in der Ausgabeeinrichtung oder über einen anderen Schalter oder Befehl. Ist dann die Verstellung und Einstellung beendet, kann dies entweder automatisch erfasst oder manuell bestätigt werden. Der Einstellmodus ist dann beendet und die Ausgabeeinrichtung wird abgeschaltet. Der Inertialwinkelsensor kann weiter betrieben werden, beispielsweise um Sensordaten für eine Steuerungseinrichtung einer anderen prothetischen Komponente bereitzustellen, beispielsweise zur Steuerung eines Prothesenkniegelenkes. Bevorzugt ist der Inertialwinkelsensor als Teil einer Steuerungseinrichtung der Protheseneinrichtung ausgebildet, so dass die Raumlagedaten des Inertialwinkelsensors nicht nur zum Erkennen und Einstellen bei Absatzhöhenänderungen oder Schuhwechsel verwendet werden, sondern auch während des Gehen als Basis für beispielsweise eine Veränderung eines Dämpfungswiderstandes in dem Knöchelgelenk und/oder dem Kniegelenk dienen.

Die Einstelleinrichtung zum manuellen Einstellen einer Orientierung eines Unterschenkelteils relativ zu einem Prothesenfuß, einer Protheseneinrichtung, einer unteren Extremität sieht vor, dass die Einstelleinrichtung einen Inertialwinkelsensor aufweist, der die Orientierung des Unterschenkelteils im Raum detektiert und mit einer Ausgabeeinrichtung gekoppelt ist, die die Orientierung des Unterschenkelteils im Raum oder das Erreichen einer vorab festgelegten Orientierung für einen Nutzer erkennbar mit einem Ausgabesignal ausgibt. Insbesondere erfolgt die Ausgabe als optisches, akustisches und/oder taktiles Ausgabesignal, wobei die Ausgabeeinrichtung in einer Variante der Erfindung mit dem Inertialwinkelsensor und einer Recheneinrichtung zum Auswerten der Inertialwinkelsensordaten und Übermittlung an die Ausgabeeinrichtung zu einem Modul zusammengefasst gekoppelt ist.

An der Einstelleinrichtung kann eine Befestigungseinrichtung zur Festlegung an einer Protheseneinrichtung angeordnet oder ausgebildet sein, beispielsweise können Formschlusselemente wie Klipse, Klettverschlussteile, Schrauben, Schnappelemente und/oder Haken oder Kraftschlusselemente wie Magnete an der jeweiligen Komponente angeordnet oder ausgebildet sein, um eine dauerhafte oder wieder lösbare und austauschbare Anbringung zu gewährleisten. Bevorzugt erfolgte die Anbringung an dem Unterschenkelteil oder einer anderen Prothesenkomponente in einer definierten Orientierung, beispielsweise entlang einer Anlagekante oder einer anderen Führung, beispielsweise an einer Schiene oder in einer Nut.

Das Verfahren zum manuellen Einstellen einer Orientierung eines Unterschenkelteils einer Protheseneinrichtung an einer unteren Extremität relativ zu einem an dem Unterschenkelteil befestigten Prothesenfuß, wobei an der Protheseneinrichtung eine Einstelleinrichtung mit einem Inertialwinkelsensor angeordnet ist, der der Orientierung des Unterschenkelteils im Raum detektiert und mit einer Ausgabeeinrichtung gekoppelt ist, sieht vor, dass eine Referenzorientierung des Unterschenkelteils im Raum für einen Nutzer eingestellt wird und das Erreichen der vorab eingestellten Referenzorientierung für einen Nutzer erkennbar mit einem Ausgabesignal ausgegeben wird. Die Referenzorientierung des Unterschenkelteils wird bevorzugt durch einen Orthopädietechniker oder einen anderen, dafür ausgebildeten Experten vorgenommen. Die Eingabe für die Referenzorientierung erfolgt bevorzugt im angelegten Zustand der Protheseneinrichtung bei einem Referenzaufbau für die Protheseneinrichtung bei einer üblichen Belastung. Eine übliche Belastung ist beispielsweise das Stehen bei einer gleichmäßigen Gewichtsbelastung auf der versorgten und unversorgten Seite. Bei einem solchen Verfahren ist es möglich, als relevante Größe die Orientierung des Unterschenkelteils im Raum zu verwenden, sodass keine Referenzpositionen für unterschiedliche Positionen für jeden Prothesenfuß eingespeichert werden müssen. Der Anwender bekommt nur ein einziges Signal, nämlich wenn die Referenzorientierung erreicht ist, gegebenenfalls mit Hinweisen, um wie viel eine Verstellung in welche Richtung notwendig ist, um die Referenzorientierung zu erreichen. Die Verstellung erfolgt manuell, insbesondere durch eine Verschwenkung um eine Knöchelgelenksachse, die senkrecht zu der Längserstreckung des Unterschenkelteils in der Frontalebene verläuft. Dadurch wird gewährleistet, dass das Unterschenkelteil nur in der Sagittalebene verschwenkt wird. Alternativ kann eine Verschwenkung an der Befestigungseinrichtung des Fußteils an dem Unterschenkelteil erfolgen, beispielsweise an dem sogenannten Pyramidenadapter, wobei dort auch eine Verschwenkung in der Frontalebene grundsätzlich möglich ist.

Die Einstellung der Orientierung des Unterschenkelteils erfolgt bevorzugt bei einer angelegten, insbesondere belasteten Protheseneinrichtung, um für den Nutzer einen bei der Benutzung gleichbleibenden Prothesenaufbau auch bei unterschiedlichen Schuhen gewährleisten zu können.

Die Einstellung kann automatisch bei jedem Prothesenfußwechsel, bei jedem Absatzhöhenwechsel oder auf ein gesondertes Aktivierungssignal hin initiiert und ausgeführt werden. Wird beispielsweise nach dem Anlegen der Protheseneinrichtung eine von der Referenzorientierung abweichende Raumlage detektiert, kann ein Hinweis an den Nutzer zur Neuorientierung oder Überprüfung ausgegeben werden und ein Einstell- oder Überprüfungsmodus eingeschaltet werden. Nach erfolgter Einstellung schaltet sich die Einstelleinrichtung bevorzugt automatisch aus, insbesondere wird die Ausgabeeinrichtung abgeschaltet, um den Energieverbrauch zu minimieren. Wird beispielsweise ein Schuh gewechselt, gibt die Ausgabeeinrichtung ein Ausgabesignal oder Feedback, dass die gegenwärtig vorhandene Unterschenkelorientierung von der Referenzorientierung abweicht. Daraufhin wird der relative Winkel zwischen dem Unterschenkel und dem Prothesenfuß manuell eingestellt, bis die Ausgabeeinrichtung das Ausgabesignal oder Feedback gibt, das die Unterschenkelorientierung der Referenzorientierung entspricht oder ausreichend nahe angenähert ist. Nach Erreichen der korrekten Position wird der Prothesenfuß in dieser Stellung fixiert, insbesondere manuell, so dass sich das proximale Anschlussmittel des Prothesenfußes, z.B. ein Pyramidenadapter, sich nicht mehr relativ zu dem Unterschenkelteil bewegt. Der Prothesenfuß selbst kann ein Gelenk aufweisen oder sich bereichsweise relativ zu dem Unterschenkelteil bewegen. Das Ende des Einstellvorganges kann über die Ausgabeeinrichtung angezeigt oder ausgegeben werden, beispielsweise nachdem automatisch erkannt wurde, dass die korrekte Einstellung vorliegt oder wenn ein entsprechendes Bestätigungssignal eingegeben worden ist.

Nachfolgend werden Ausführungsbeispiele der Erfindung anhand der beigefügten Figuren näher erläutert. Es zeigen:
- Figur 1 -: eine schematische Darstellung eines Einstellvorganges;
- Figur 2 -: eine Protheseneinrichtung mit unterschiedlichen Schuhen;
- Figur 3 -: eine schematische Darstellung einer Protheseneinrichtung mit Unterschenkelschaft;
- Figur 4 -: eine Variante der Figur 3 mit Prothesenkniegelenk und Oberschenkelschaft;
- Figur 5 -: eine schematische Darstellung mit einer separaten Ausgabeeinrichtung; sowie
- Figur 6 -: eine Variante mit einer integrierten Ausgabeeinrichtung.

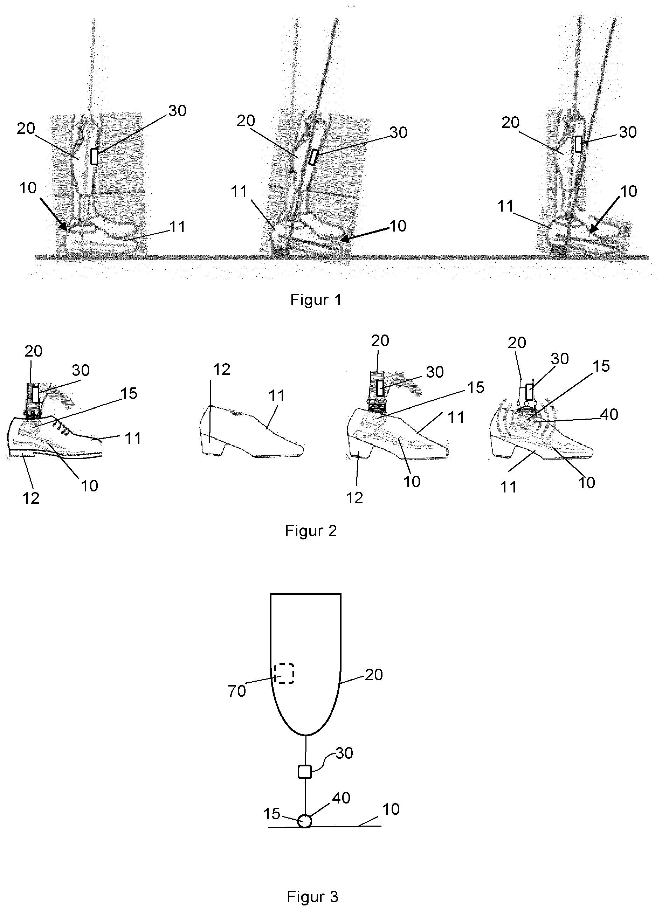

Die Figur 1 zeigt drei Positionen oder Zustände, in denen sich eine Protheseneinrichtung während der Benutzung befinden kann. In der linken Darstellung der Figur 1 ist die Protheseneinrichtung mit einem Prothesenfuß 10 und einem Unterschenkelteil 20 gezeigt. An dem proximalen Ende des Unterschenkelteils 20 ist ein Prothesenkniegelenk angeordnet, dass mit einem nicht dargestellten Oberschenkelschaft verbunden ist. Über den Oberschenkelschaft wird die Protheseneinrichtung an einem Oberschenkelstumpf festgelegt. In dem dargestellten Ausführungsbeispiel ist an dem Unterschenkelteil 20 ein Inertialwinkelsensor 30, der auch als inertiale Messeinheit oder IMU bezeichnet wird, angeordnet, der als eine Baugruppe aus einem oder mehreren Gyroskopen, ggf. mit Beschleunigungssensoren ergänzt, aufgebaut sein kann. Die in der linken Darstellung der Figur 1 gezeigte Orientierung wird als eine Referenzorientierung gespeichert, wobei sich als Bezugsgrößen die jeweiligen Längserstreckungen des Prothesenfußes 10 und des Unterschenkelteils 20 anbieten. Die gespeicherte Referenzorientierung ist der sogenannte Referenzaufbau der Protheseneinrichtung, der durch einen Orthopädietechniker eingestellt, eingespeichert und dokumentiert wird. Die Speicherung kann in einer Speichereinrichtung erfolgen, die Teil einer Steuerungseinrichtung zum Steuern einer Dämpfereinrichtung in dem Prothesenkniegelenk sein kann. Ebenfalls kann der Inertialwinkelsensor 30 Teil der Steuerungseinrichtung für das Prothesenkniegelenk sein. Der Prothesenaufbau ist die räumliche Zuordnung der einzelnen Prothesenkomponenten zueinander. In der Referenzeinstellung wird angestrebt, dass alle Prothesenkomponenten optimal zueinander ausgerichtet sind, damit der Prothesennutzer den größtmöglichen Nutzen aus der Protheseneinrichtung ziehen kann. Da in der Regel die Protheseneinrichtung mit einem Schuh 11getragen wird, ist es notwendig, den Prothesenaufbau mit einem angelegten Schuh 11 einzustellen. Der Schuh 11 ist in der Regel ein Modell, das von dem Nutzer üblicherweise getragen wird. Wird das Schuhmodell gewechselt und hat der Schuh 11 eine andere Absatzhöhe, wie dies in der mittleren Darstellung der Figur 1 dargestellt ist, verändert sich der Prothesenaufbau und insbesondere die Orientierung des Unterschenkelteils 20. In der mittleren Darstellung der Figur 1 ist zu erkennen, dass die Längserstreckung des Unterschenkelteils 20 aufgrund der unterschiedlichen Absatzhöhe nach vorne geneigt ist, ebenso ist die Neigung des Prothesenfußes 10 verändert. Der Inertialwinkelsensor 30 oder die IMU 30 detektieren entweder nach der Aktivierung eines Überprüfungsmodus durch den Nutzer oder automatisch die Neigung und Orientierung des Unterschenkelteils 30 im Raum. Da die Orientierung des Unterschenkelteils 30 nicht mehr der Referenzorientierung entspricht, wird über eine nicht dargestellte Ausgabeeinrichtung das Signal ausgegeben, dass die Ausrichtung und der Prothesenaufbau nicht mehr korrekt sind. Daraufhin wird beispielsweise eine Verriegelung einer Schwenkachse, um die der Prothesenfuß 10 relativ zu dem Unterschenkelteil 20 verschwenkt werden kann, entsperrt oder gelöst und das Unterschenkelteil 20 so lange verschwenkt, bis die Referenzorientierung wieder eingenommen worden ist. Sobald dies der Fall ist, gibt die Ausgabeeinrichtung ein entsprechendes Signal an den Nutzer, der die Verriegelungseinrichtung wieder aktivieren und die Schwenkachse sperren kann. Die korrekte Raumlage wird über die IMU oder den Inertialwinkelsensor 30 detektiert und durch ein optisches, akustisches und/oder taktiles Signal angezeigt. Alternativ kann über die Ausgabeeinrichtung der jeweils eingenommene Raumlagewinkel oder die Entfernung zu dem Referenzwinkel bzw. zu der Referenzorientierung angezeigt werden. In der rechten Darstellung ist das Unterschenkelteil 20 wieder in der ursprünglichen Referenzorientierung, was durch die gestrichelte Linie angedeutet ist.

In der Figur 2 sind die relevanten Komponenten der Protheseneinrichtung in Einzeldarstellung gezeigt. Die linke Darstellung zeigt einen Prothesenfuß 10 in einem Schuh 11 mit einem Absatz 12. Der Prothesenfuß 10 weist eine Verschwenkeinrichtung 15 auf, um die das Unterschenkelteil 20 in Gestalt eines Unterschenkelrohres um eine Schwenkachse verschwenkt werden kann. An dem Unterschenkelteil 20 ist ein Inertialwinkelsensor 30 befestigt. Die zweite Darstellung von links zeigt einen alternativen Schuh 11 mit einem höheren Absatz 12. In der dritten Darstellung von links ist der Prothesenfuß 10 in den alternativen Schuh 11 eingeführt. Aufgrund der unterschiedlichen Sprengung zwischen den beiden Schuhmodellen ist es notwendig, das Unterschenkelteil 20 entgegen der Gegenrichtung, also nach hinten zu verschwenken. Dazu wird das Unterschenkelteil 20 in Pfeilrichtung nach hinten verschwenkt und der Verschwenkvorgang bzw. die Raumlagenorientierung des Unterschenkelteils 20 über den Inertialwinkelsensor 30 überprüft. Sobald die korrekte Ausrichtung des Unterschenkelteils 20 im Raum erreicht ist, insbesondere wenn der Patient sowohl die versorgte Seite als auch die unversorgte Seite gleichmäßig belastet, wird über eine Ausgabeeinrichtung 40 ein optisches, akustisches und/oder taktiles Signal ausgegeben, das anzeigt, dass die korrekte Orientierung erreicht ist. Der Nutzer verriegelt dann die Verschwenkeinrichtung 15 gegen ein ungewolltes Verlagern des Prothesenfußes 10 relativ zu dem Unterschenkelteil 30. Anschließend kann die Stromversorgung zu der Ausgabeeinrichtung 40 unterbrochen werden, um Energie zu sparen. Der Inertialwinkelsensor 30 kann zur Bereitstellung von Sensordaten weiter genutzt werden.

**In** der Figur 3 ist in einer schematischen Darstellung die Protheseneinrichtung mit dem Prothesenfuß 10 dem Unterschenkelteil 20 sowie dem daran befestigten Inertialwinkelsensor 30 gezeigt. Der Prothesenfuß 10 ist über die Verschwenkeinrichtung 15 um eine Achse verschwenkbar an dem Unterschenkelteil 20 gelagert. Das Unterschenkelteil 20 weist einen Unterschenkelschaft sowie ein Unterschenkelrohr auf, an dem der Inertialwinkelsensor 30 entweder dauerhaft oder abnehmbar festgelegt ist. An dem Unterschenkelschaft ist darüber hinaus eine Befestigungseinrichtung 70 vorgesehen, an der die nicht dargestellte Ausgabeeinrichtung 40 oder ein Modul aus dem Inertialwinkelsensor 30 und der Ausgabeeinrichtung 40 festgelegt werden kann. Das Modul, die Ausgabeeinrichtung 40 und/oder der Inertialwinkelsensor 30 sind bevorzugt zerstörungsfrei lösbar an der Befestigungseinrichtung 70 festlegbar und anbringbar ausgestaltet. Die Festlegung kann über Formschlusselemente wie Schrauben, Bolzen, Haken oder Clips-Elemente oder kraftschlüssig mittels Magneten oder mit einer Kombination aus Formschlusselementen und Kraftschlusselementen erfolgen. Die Anordnung des Inertialwinkelsensor 30 oder der IMU kann entweder integriert in oder lösbar befestigt an einem Teil des Knöchelgelenks erfolgen, der fest mit dem Unterschenkelteil 20 verbunden ist. Ebenfalls ist es möglich, den Sensor 30 an einem Strukturteil des Unterschenkelteils 20 anzuordnen, beispielsweise dem Unterschenkelrohr oder in dem Unterschenkelschaft.

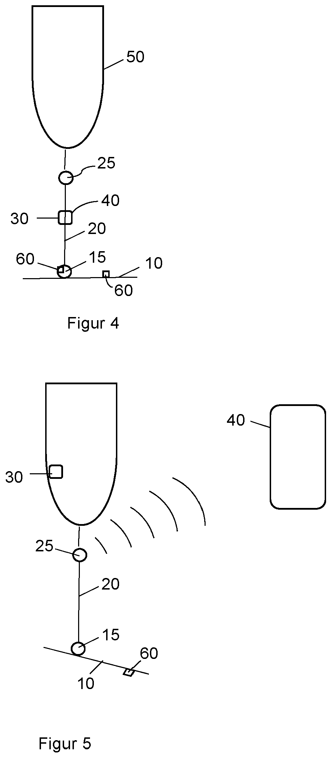

In der Figur 4 ist eine Protheseneinrichtung schematisch dargestellt, bei der eine proximale Prothesenkomponente 50 an dem Unterschenkelteil 20 angeordnet ist. Die proximale Prothesenkomponente 50 ist beispielsweise ein Oberschenkelschaft mit einem Verbindungsrohr zu einem Prothesenkniegelenk 25. In dem dargestellten Ausführungsbeispiel ist der Inertialwinkelsensor 30 wieder an dem Unterschenkelteil 20 angeordnet, alternativ kann der Inertialwinkelsensor 30 und gegebenenfalls auch die Ausgabeeinrichtung 40 integriert oder lösbar befestigt an dem Kniegelenk 25 oder dem Oberschenkelschaft oder einem Verbindungsteil zwischen dem Oberschenkelschaft und dem Prothesenkniegelenk angeordnet sein. Ebenfalls kann der Inertialwinkelsensor 30 an einem Oberteil eines Prothesenkniegelenkes angeordnet sein. In Verbindung mit einem Winkelsensor, der den Winkel zwischen dem Unterschenkelteil 20 und der proximalen Komponente 50 erfasst, kann aus der Raumlage der proximalen Komponente 50 die Raumlage des Unterschenkelteils 20 detektiert werden. In dem Ausführungsbeispiel der Figur 4 sind darüber hinaus Belastungssensoren 60 vorgesehen, die an dem Prothesenfuß 10 und der Verschwenkeinrichtung 15 angeordnet sind. Die Belastungssensoren 60 können beispielsweise Axialkraftsensoren, Drucksensoren und/oder Momentensensoren sein, um die jeweilige Belastung auf die Protheseneinrichtung zu erfassen. Die Belastungssensoren 60 oder der Belastungssensor 60 sind/ist mit einer Steuerungseinrichtung gekoppelt, die auch mit dem Inertialwinkelsensor 30 gekoppelt ist. So kann beispielsweise erkannt werden, ob eine Einstellung des Prothesenaufbaus bei einer belasteten Protheseneinrichtung erfolgt oder nicht. Die Ausgabeeinrichtung 40 ist in dem Ausführungsbeispiel gemäß Figur 4 mit dem Inertialwinkelsensor 30 zu einem Modul zusammengefasst und an dem Unterschenkelteil 20 festgelegt.

In der Figur 5 ist die Ausgabeeinrichtung 40 von dem Inertialwinkelsensor 30 getrennt und räumlich separiert ausgebildet, beispielsweise in Gestalt eines Mobiltelefones, an das drahtlos entsprechende Daten von dem Inertialwinkelsensor 30, gegebenenfalls über eine separate Sendeeinrichtung, übermittelt werden. Die Datenübertragung von der Sensorik zu der Ausgabeeinrichtung 40 kann über Funk, WLAN, Bluetooth, NFC oder andere Übertragungswege erfolgen. Die Ausgabeeinrichtung kann neben einer optischen Anzeige eine akustische Rückkopplung oder ein Vibrationssignal ausgeben, um den Nutzer über die korrekte Einstellung nach einem Absatz Höhenwechseln zu informieren.

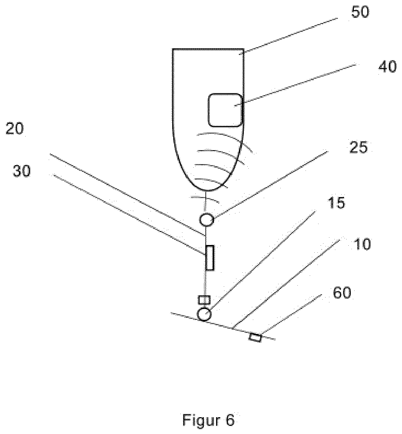

In der Figur 6 ist eine weitere Variante der Erfindung dargestellt, bei der die Ausgabeeinrichtung 40 an dem Prothesenschaft 50 als proximale Komponente angeordnet ist. Die Ausgabeeinrichtung 40 ist in dem Prothesenschaft 50 integriert. Der Inertialwinkelsensor 30 oder die IMU ist an dem Unterschenkelteil 20 angeordnet. Die Übertragung von dem Inertialwinkelsensor 30 zu der Ausgabeeinrichtung 40 erfolgt drahtlos. Nach einer manuellen Entriegelung des Prothesenfußes 10 relativ zu dem Unterschenkelteil 20 wird beispielsweise der Prothesenfuß 10 mit einem Schuh, insbesondere einem Schuh mit einer zu einem vorher angepassten Schuh abweichenden Absatzhöhe auf den Boden gesetzt. Der Kontakt des Prothesenfußes 10 mit dem Boden wird über den Kraftsensor 60 ermittelt. Das Unterschenkelteil 20 wird um das Knöchelgelenk 15 solange verschwenkt, bis die vorher eingestellte Referenzorientierung des Unterschenkelteils 20 im Raum erreicht ist. Dazu kann beispielsweise vorgesehen sein, dass sich das Unterschenkelteil 20 innerhalb der Sagittalebene befindet oder aber innerhalb eines festgelegten Winkelbereiches medial und lateral der Sagittalebene. Bei Erreichen der Referenzorientierung des Unterschenkelteils 20 wird von der Ausgabeeinrichtung 40 ein optisches, akustisches oder taktiles Signal ausgegeben, das das Erreichen der gewünschten Stellung erfolgt ist.

## Patentansprüche

1. Protheseneinrichtung für eine untere Extremität mit einem Prothesenfuß (10) und einem an dem Prothesenfuß (10) befestigten Unterschenkelteil (20) sowie einer Einrichtung zum manuellen Einstellen einer Orientierung des Unterschenkelteils (20) relativ zu dem Prothesenfuß (10), **dadurch gekennzeichnet, dass** an der Protheseneinrichtung ein Inertialwinkelsensor (30) angeordnet ist, der die Orientierung des Unterschenkelteils (20) im Raum detektiert und mit einer Ausgabeeinrichtung (40) gekoppelt ist, die die Orientierung des Unterschenkelteils (20) im Raum oder das Erreichen einer vorab festgelegten Orientierung für einen Nutzer erkennbar mit einem Ausgabesignal ausgibt.

2. Protheseneinrichtung nach Anspruch 1, **dadurch gekennzeichnet, dass** der Inertialwinkelsensor (30) und die Ausgabeeinrichtung (40) als Modul zusammengefasst in der Protheseneinrichtung integriert oder lösbar daran befestigt sind.

3. Protheseneinrichtung nach Anspruch 1 oder 2, **dadurch gekennzeichnet, dass** der Inertialwinkelsensor (30) an dem Unterschenkelteil (20) angeordnet ist oder die Protheseneinrichtung eine proximal zu dem Unterschenkelteil (20) angeordnete Prothesenkomponente (50) aufweist, an der der Inertialwinkelsensor (30) angeordnet ist.

4. Protheseneinrichtung nach einem der voranstehenden Ansprüche, **dadurch gekennzeichnet, dass** ein Belastungssensor (60) an der Protheseneinrichtung angeordnet und mit der Ausgabeeinrichtung (40) dergestalt gekoppelt ist, dass das Ausgabesignal bei einer detektierten Belastung ausgegeben wird.

5. Protheseneinrichtung nach Anspruch 4, **dadurch gekennzeichnet, dass** der Belastungssensor (60) als Axialkraftsensor, Drucksensor oder Momentensensor ausgebildet ist.

6. Protheseneinrichtung nach einem der voranstehenden Ansprüche, **dadurch gekennzeichnet, dass** die Ausgabeeinrichtung (40) zur Ausgabe eines optischen, akustischen und/oder taktilen Ausgabesignals ausgebildet ist.

7. Protheseneinrichtung nach einem der voranstehenden Ansprüche, **dadurch gekennzeichnet, dass** der Prothesenfuß (10) in der Sagittalebene verschwenkbar gelagert ist.

8. Protheseneinrichtung nach einem der voranstehenden Ansprüche, **gekennzeichnet durch** eine Abschalteinrichtung, die den Inertialwinkelsensor (30) und/oder die Ausgabeeinrichtung (40) oder die Verbindung von dem Inertialwinkelsensor (30) zu der Ausgabeeinrichtung (40) nach Erreichen der vorab festgelegten Orientierung abschaltet.

9. Einstelleinrichtung zum manuellen Einstellen einer Orientierung eines Unterschenkelteils (20) relativ zu einem Prothesenfuß (10) einer Protheseneinrichtung einer unteren Extremität, **dadurch gekennzeichnet, dass** die Einstelleinrichtung einen Inertialwinkelsensor (30) aufweist, der die Orientierung des Unterschenkelteils (20) im Raum detektiert und mit einer Ausgabeeinrichtung (40) gekoppelt ist, die die Orientierung des Unterschenkelteils (20) im Raum oder das Erreichen einer vorab festgelegten Orientierung für einen Nutzer erkennbar mit einem Ausgabesignal ausgibt.

10. Einstelleinrichtung nach Anspruch 9, **dadurch gekennzeichnet, dass** die Ausgabeeinrichtung (40) zur Ausgabe eines optischen, akustischen und/oder taktilen Ausgabesignals ausgebildet ist.

11. Einstelleinrichtung nach Anspruch 9 oder 10, **dadurch gekennzeichnet, dass** eine Befestigungseinrichtung (70) zur Festlegung an einer Protheseneinrichtung an der Einstelleinrichtung angeordnet oder ausgebildet ist.

12. Verfahren zum manuellen Einstellen einer Orientierung eines Unterschenkelteils (20) einer Protheseneinrichtung einer unteren Extremität relativ zu einem an dem Unterschenkelteil (20) befestigten Prothesenfuß (10), wobei an der Protheseneinrichtung eine Einstelleinrichtung mit einem Inertialwinkelsensor (30) angeordnet ist, der die Orientierung des Unterschenkelteils (20) im Raum detektiert und mit einer Ausgabeeinrichtung (40) gekoppelt ist, **dadurch gekennzeichnet, dass** eine Referenzorientierung des Unterschenkelteils (20) im Raum für einen Nutzer eingestellt wird und das Erreichen der vorab eingestellten Referenzorientierung für einen Nutzer erkennbar mit einem Ausgabesignal ausgegeben wird.

13. Verfahren nach Anspruch 12, **dadurch gekennzeichnet, dass** die Einstellung bei einer angelegten, insbesondere belasteten Protheseneinrichtung durchgeführt wird.

14. Verfahren nach Anspruch 12 oder 13, **dadurch gekennzeichnet, dass** die Einstellung automatisch bei jedem Prothesenfußwechsel, Absatzhöhenwechsel oder auf ein Aktivierungssignal ausgeführt wird.

## Claims

1. A prosthetic device for a lower extremity, comprising a prosthetic foot (10) and a lower-leg part (20) fastened to the prosthetic foot (10), and a device for manually adjusting an orientation of the lower-leg part (20) relative to the prosthetic foot (10), **characterized in that** an inertial angle sensor (30) is arranged on the prosthetic device, serves to detect the orientation of the lower-leg part (20) in space and is coupled to an output device (40) which outputs, in a manner identifiable by a user by way of an output signal, the orientation of the lower-leg part (20) in space or the attainment of an orientation defined in advance.

2. The prosthetic device as claimed in claim 1, **characterized in that** the inertial angle sensor (30) and the output device (40) are combined as a module and integrated in the prosthetic device or detachably fastened thereto.

3. The prosthetic device as claimed in claim 1 or 2, **characterized in that** the inertial angle sensor (30) is arranged on the lower-leg part (20) or the prosthetic device has a prosthesis component (50) arranged proximal to the lower-leg part (20), the inertial angle sensor (30) being arranged on said prosthesis component (50).

4. The prosthetic device as claimed in any one of the preceding claims, **characterized in that** a load sensor (60) is arranged on the prosthetic device and is coupled to the output device (40) in such a way that the output signal is output if a load is detected.

5. The prosthetic device as claimed in claim 4, **characterized in that** the load sensor (60) is designed as an axial force sensor, pressure sensor or torque sensor.

6. The prosthetic device as claimed in any one of the preceding claims, **characterized in that** the output device (40) is designed to output an optical, acoustic and/or tactile output signal.

7. The prosthetic device as claimed in any one of the preceding claims, **characterized in that** the prosthetic foot (10) is mounted so as to be pivotable in the sagittal plane.

8. The prosthetic device as claimed in any one of the preceding claims, **characterized by** a deactivation device, which deactivates the inertial angle sensor (30) and/or the output device (40) or the connection between the inertial angle sensor (30) and the output device (40) after the orientation defined in advance has been attained.

9. An adjustment device for manually adjusting an orientation of a lower-leg part (20) relative to a prosthetic foot (10) of a prosthetic device of a lower extremity, **characterized in that** the adjustment device comprises an inertial angle sensor (30) which detects the orientation of the lower-leg part (20) in space and which is coupled to an output device (40) which outputs, in a manner identifiable by a user by way of an output signal, the orientation of the lower-leg part (20) in space or the attainment of an orientation defined in advance.

10. The adjustment device as claimed in claim 9, **characterized in that** the output device (40) is designed to output an optical, acoustic and/or tactile output signal.

11. The adjustment device as claimed in claim 9 or 10, **characterized in that** a fastening device (70) for securing to a prosthetic device is arranged or formed on the adjustment device.

12. A method for manually adjusting an orientation of a lower-leg part (20) of a prosthetic device of a lower extremity relative to a prosthetic foot (10) fastened to the lower-leg part (20), wherein an adjustment device with an inertial angle sensor (30) is arranged on the prosthetic device, the inertial angle sensor detecting the orientation of the lower-leg part (20) in space and being coupled to an output device (40), **characterized in that** a reference orientation of the lower-leg part (20) in space is set for a user and the attainment of the reference orientation set in advance is output in a manner identifiable by a user by way of an output signal.

13. The method as claimed in claim 12, **characterized in that** the adjustment is performed in the case of an applied, more particularly loaded prosthetic device.

14. The method as claimed in claim 12 or 13, **characterized in that** the adjustment is carried out automatically for each change in prosthetic foot, for each change in heel height or following an activation signal.

## Revendications

1. Dispositif prothétique pour un membre inférieur, comprenant un pied prothétique (10) et une partie de jambe (20) fixée au pied prothétique (10) ainsi qu'un dispositif de réglage manuel d'une orientation de la partie de jambe (20) par rapport au pied prothétique (10),
**caractérisé en ce qu'**un capteur d'angle inertiel (30) est disposé sur le dispositif prothétique, lequel détecte l'orientation de la partie de jambe (20) dans l'espace et est couplé à un dispositif de sortie (40) qui émet un signal de sortie indiquant, de manière reconnaissable par un utilisateur, l'orientation de la partie de jambe (20) dans l'espace ou le fait qu'une orientation définie préalablement a été atteinte.

2. Dispositif prothétique selon la revendication 1,
**caractérisé en ce que** le capteur d'angle inertiel (30) et le dispositif de sortie (40) sont intégrés dans le dispositif prothétique ou y sont fixés de manière amovible, en étant réunis en un module.

3. Dispositif prothétique selon la revendication 1 ou 2,
**caractérisé en ce que** le capteur d'angle inertiel (30) est disposé sur la partie de jambe (20), ou le dispositif prothétique comprend un composant prothétique (50) disposé du côté proximal par rapport à la partie de jambe (20), sur lequel est disposé le capteur d'angle inertiel (30).

4. Dispositif prothétique selon l'une des revendications précédentes,
**caractérisé en ce qu'**un capteur de charge (60) est disposé sur le dispositif prothétique et est couplé au dispositif de sortie (40) de telle sorte que le signal de sortie est émis lorsqu'une charge est détectée.

5. Dispositif prothétique selon la revendication 4,
**caractérisé en ce que** le capteur de charge (60) est conçu comme un capteur de force axiale, un capteur de pression ou un capteur de couple.

6. Dispositif prothétique selon l'une des revendications précédentes,
**caractérisé en ce que** le dispositif de sortie (40) est conçu pour émettre un signal de sortie optique, acoustique et/ou tactile.

7. Dispositif prothétique selon l'une des revendications précédentes,
**caractérisé en ce que** le pied prothétique (10) est monté de manière à pouvoir pivoter dans le plan sagittal.

8. Dispositif prothétique selon l'une des revendications précédentes,
**caractérisé par** un dispositif de coupure qui, une fois que l'orientation définie préalablement est atteinte, coupe le capteur d'angle inertiel (30) et/ou le dispositif de sortie (40) ou la liaison entre le capteur d'angle inertiel (30) et le dispositif de sortie (40).

9. Dispositif de réglage manuel d'une orientation d'une partie de jambe (20) par rapport à un pied prothétique (10) d'un dispositif prothétique d'un membre inférieur,
**caractérisé en ce que** le dispositif de réglage présente un capteur d'angle inertiel (30) qui détecte l'orientation de la partie de jambe (20) dans l'espace et qui est couplé à un dispositif de sortie (40) qui émet un signal de sortie indiquant, de manière reconnaissable par un utilisateur, l'orientation de la partie de jambe (20) dans l'espace ou le fait qu'une orientation définie préalablement a été atteinte.

10. Dispositif de réglage selon la revendication 9,
**caractérisé en ce que** le dispositif de sortie (40) est conçu pour émettre un signal de sortie optique, acoustique et/ou tactile.

11. Dispositif de réglage selon la revendication 9 ou 10,
**caractérisé en ce qu'**un dispositif de fixation (70) destiné à être fixé à un dispositif prothétique est disposé ou formé sur le dispositif de réglage.

12. Procédé de réglage manuel d'une orientation d'une partie de jambe (20) d'un dispositif prothétique d'un membre inférieur par rapport à un pied prothétique (10) fixé à la partie de jambe (20), un dispositif de réglage étant disposé sur le dispositif prothétique et étant muni d'un capteur d'angle inertiel (30) qui détecte l'orientation de la partie de jambe (20) dans l'espace et qui est couplé à un dispositif de sortie (40),
**caractérisé en ce qu'**une orientation de référence de la partie de jambe (20) dans l'espace est réglée pour un utilisateur, et un signal de sortie est émis qui indique, de manière reconnaissable par un utilisateur, le fait que l'orientation de référence réglée préalablement est atteinte.

13. Procédé selon la revendication 12,
**caractérisé en ce que** le réglage est effectué lorsque le dispositif prothétique est mis en place, en particulier lorsqu'il est sous charge.

14. Procédé selon la revendication 12 ou 13,
**caractérisé en ce que** le réglage est effectué automatiquement à chaque changement du pied prothétique, à chaque changement de la hauteur du talon ou en réponse à un signal d'activation.
